**Europäisches Patentamt**

**European Patent Office**

**Office européen des brevets**

(19)

(11) Veröffentlichungsnummer: **0 020 990**
**B1**

(12) # EUROPÄISCHE PATENTSCHRIFT

(45) Veröffentlichungstag der Patentschrift:
**12.09.84**

(51) Int. Cl.³: **C 07 C 69/82**, C 07 C 67/39,
C 07 C 67/48, B 01 J 23/90

(21) Anmeldenummer: **80102628.7**

(22) Anmeldetag: **12.05.80**

(54) Verfahren zur Gewinnung und Wiederverwendung von Oxidationskatalysator im Witten-DMT-Prozess.

(30) Priorität: **12.06.79 DE 2923681**

(43) Veröffentlichungstag der Anmeldung:
**07.01.81 Patentblatt 81/1**

(45) Bekanntmachung des Hinweises auf die Patenterteilung:
**12.09.84 Patentblatt 84/37**

(84) Benannte Vertragsstaaten:
**AT BE DE FR GB IT NL**

(56) Entgegenhaltungen:
**DE - A - 2 525 135**

**CHEMICAL ABSTRACTS, Band 89, Nr. 15, 9. Oktober
1978, Seite 567, Zusammenfassung 129276m,
COLUMBUS, OHIO (US)**

(73) Patentinhaber: **DYNAMIT NOBEL
AKTIENGESELLSCHAFT, Postfach 1209,
D-5210 Troisdorf, Bez. Köln (DE)**

(72) Erfinder: **Bünger, Heinrich, Dr., Fliederweg 11,
D-5200 Siegburg (DE)**
Erfinder: **Cordes, Rudolf, Dr., Kasseler Weg 3,
D-5216 Niederkassel (DE)**
Erfinder: **Hoffmann, Gerhart, Dr., Porzer Strasse 1,
D-5216 Niederkassel (DE)**

**Beschreibung**

Die Erfindung betrifft ein verbessertes Verfahren zur Gewinnung und Wiederverwendung von Schwermetalloxidationskatalysator durch Extraktion von hochsiedenden Destillationsrückständen, die bei der Oxidation von p-Xylol und/oder p-Toluylsäuremethylester enthaltenden Gemischen in flüssiger Phase mit Sauerstoff oder einem Sauerstoff enthaltenden Gas bei erhöhtem Druck und erhöhter Temperatur in Gegenwart von gelöstem Schwermetalloxidationskatalysator, anschliessenden Veresterung des Oxidationsprodukts mit Methanol bei erhöhtem Druck und erhöhter Temperatur und destillativen Auftrennung des Veresterungsprodukts in eine Roh-DMT-Fraktion, eine p-toluylsäuremethylesterreiche Fraktion und einen hochsiedenden Destillationsrückstand anfallen, mit Wasser oder verdünnten wässrigen Lösungen niedermolekularer aliphatischer Monocarbonsäuren oder Alkohole und Rückführung des Extraktes, in die Oxidation gegebenenfalls nach Einengen.

Dimethylterephthalat (DMT) wird als Rohstoff zur Herstellung von Polyester durch Umsetzung mit Ethylenglykol oder Tetramethylenglykol für Fasern, Filaments, Filme oder Formteile benötigt. Es wird in zahlreichen grosstechnischen Anlagen nach dem Witten-DMT-Verfahren (vgl. DE-PS 1 041 945) hergestellt.

Dabei wird ein Gemisch von p-Xylol (PX) und p-Toluylsäuremethylester (PTE) in Abwesenheit von Lösungsmitteln und Halogenverbindungen in der flüssigen Phase bei erhöhtem Druck und erhöhter Temperatur mit Sauerstoff oder einem Sauerstoff enthaltenden Gas in Gegenwart von gelöstem Schwermetalloxidationskatalysator, insbesondere eines Gemisches von Kobalt und Mangan, oxidiert (vgl. DE-AS 2 010 137).

Anschliessend wird das erhaltene Reaktionsgemisch, das überwiegend aus Monomethylterephthalat (MMT) und aus p-Toluylsäure (PTS) besteht, mit Methanol verestert. Das Veresterungsprodukt wird destillativ aufgetrennt in eine PTE-Fraktion, eine DMT-Fraktion und einen hochsiedenden teerartigen Rückstand. Die PTE-Fraktion wird in die Oxidation zurückgeführt. Der hochsiedende teerartige Destillationsrückstand enthält unter anderem sämtliche Bestandteile des Katalysators, z.B. Kobalt und Mangan.

Es ist vorteilhaft, den Oxidationskatalysator aus dem teerartigen Destillationsrückstand zurückzugewinnen und erneut zur Oxidation von PX und PTE einzusetzen.

Es ist möglich, den teerartigen Destillationsrückstand zu verbrennen, dabei die Bestandteile des Schwermetalloxidationskatalysators in Oxide umzuwandeln und diese als Asche zu gewinnen. Die Asche kann z.B. mit Zyklonen oder mit Elektrofiltern aus der Verbrennungsabluft gewonnen werden.

Bei dieser Arbeitsweise treten Kobaltverluste durch flüchtige Kobaltverbindungen auf. Ausserdem kann die Asche nicht direkt als Oxidationskatalysator wiederverwendet werden. Sie muss zuvor in Mineralsäuren gelöst werden, anschliessend müssen die Schwermetalle ausgefällt werden. Die so erhaltenen Niederschläge enthalten neben den als Oxidationskatalysatoren brauchbaren Schwermetallen unerwünschte Verunreinigungen, z.B. Eisen und Chrom, die aus einer Korrosion des Anlagenwerkstoffes herrühren, und zusätzlich Vanadium, falls die teerartigen Destillationsrückstände mit Hilfe von schwerem Heizöl verbrannt werden.

Weiterhin wurde vorgeschlagen, den Oxidationskatalysator aus dem teerartigen Destillationsrückstand, der bei der DMT-Herstellung nach dem Witten-Verfahren anfällt, durch Flüssig-Flüssig-Extraktion zu gewinnen und den katalysatorhaltigen Extrakt direkt in die Oxidationsstufe zurückzuführen. Als Extraktionsmittel wurden vorgeschlagen: Wasser, Wasser unter gleichzeitigem Einleiten von Luft, Gemische aus Wasser und niederen aliphatischen Alkoholen sowie Gemische aus Wasser und niederen aliphatischen Fettsäuren (vgl. DE-AS 2 531 106).

Ferner ist bekannt, dass Trimellitsäuretrimethylester in Konzentrationen über 0,8 Gew.-%, bezogen auf das Oxidat, die Oxidationsgeschwindigkeit verringert (CA 89 129 276 n).

Diese Extraktionsverfahren haben den Nachteil, dass die katalytische Selektivität der so erhaltenen schwermetallhaltigen Extrakte, die im folgenden als «Regeneratkatalysatorlösungen» bezeichnet werden, geringer ist als die gleichkonzentrierter Lösungen frischen Oxidationskatalysators und dass die katalytische Selektivität der Regeneratkatalysatorlösungen nicht konstant ist, sondern trotz konstanten Metallgehalts sehr unterschiedliche Werte annehmen kann.

Die geringere und inkonstante katalytische Selektivität bewirkt, dass die Ausbeute an DMT niedriger ist als bei Verwendung frischen Oxidationskatalysators und dass die Verbrennungsverluste höher sind, insbesondere durch erhöhte Bildung von Kohlenmonoxid und Kohlendioxid.

Insbesondere die unerklärliche und nicht vorhersehbare Inkonstanz der Selektivität bewirkte, dass Regeneratkatalysatorlösung bisher in technischen Oxidationsanlagen grosse Schwierigkeiten verursachte.

Zur Erhöhung und Stabilisierung der Selektivität von Regeneratkatalysator wurde in der DE-OS 2 525 135 vorgeschlagen, entweder den teerartigen Destillationsrückstand vor der Flüssig-Flüssig-Extraktion bei erhöhter Temperatur unter bestimmten Bedingungen mit Methanol zu behandeln und erst danach die Schwermetalle, z.B. Kobalt und Mangan, zu extrahieren oder zunächst die Schwermetalle aus diesem teerartigen Destillationsrückstand zu extrahieren und danach den Extrakt bei erhöhter Temperatur unter bestimmten Bedingungen mit Methanol zu behandeln, erforderlichenfalls nach vorheriger Entfernung eines Teils oder der Gesamtmenge des Extraktionslösungsmittels. Diese Arbeitsweise zur Aktivierung des Regeneratkatalysators benötigt spezielle Apparaturen, um einen innigen Kontakt des Rückstands oder des Extraktes mit dem Methanol zu ermöglichen. Weil sie die Handhabung erheblicher Mengen Methanol bei erhöhter Temperatur erfordert, wird der Energieverbrauch erhöht, und es treten Methanolverluste auf.

Eine Aufgabe der vorliegenden Erfindung besteht deshalb in der Entwicklung eines Verfahrens zur Rückgewinnung von Oxidationskatalysator aus dem katalysatorhaltigen Destillationsrückstand, der bei der Herstellung von DMT nach dem Witten-Verfahren anfällt und zur Wiederverwendung des rückgewonnenen Katalysators zur Oxidation von PX und/oder PTE nach dem Witten-Verfahren, ohne dass die Selektivität der Oxidation beeinträchtigt wird, ohne dass mehr Kohlendioxid und Kohlenmonoxid entstehen und ohne dass der Extrakt oder der noch nicht extrahierte Rückstand mit Methanol behandelt werden.

Es ist notwendig, möglichst geringe Katalysatormengen in die Oxidation einzusetzen, um die Katalysatorkosten niedrigzuhalten und um Störungen in der nach der Oxidation notwendigen Veresterung zu vermeiden. Zu hohe Oxidationskatalysatorkonzentrationen führen in den Veresterungskolonnen zu Verstopfungen durch Abscheidung von Katalysatormetall. Diese Verstopfungen werden vermieden, und die Kosten der Rückgewinnung und die bei jeder Rückgewinnung unvermeidlichen Verluste werden verringert, wenn möglichst geringe Katalysatormengen in die Oxidation eingesetzt werden. Die Dosierung geringer Mengen Regeneratkatalysator war jedoch bisher ohne Verringerung der DMT-Ausbeute nicht möglich, da die Selektivität der Regeneratkatalysatorlösungen aus bisher unerklärlichen Gründen trotz konstanten Metallgehalts nicht konstant ist. Eine weitere Aufgabe der vorliegenden Erfindung besteht deshalb in der Entwicklung eines Verfahrens, das es ermöglicht, den rückgewonnenen Oxidationskatalysator in möglichst geringen Mengen in die Oxidation einzusetzen, ohne dass die Selektivität der Oxidation beeinträchtigt wird.

Weitere Aufgaben und Vorteile ergeben sich aus der Beschreibung.

Unsere Untersuchungen haben überraschend gezeigt, dass der katalysatorhaltige Extrakt aus dem nach dem Witten-Verfahren anfallenden katalysatorhaltigen Destillationsrückstand wechselnde Mengen Trimellitsäure (TMS) und Trimellitsäuremonomethylester (TMME) enthält, dass diese Verbindungen die geringste zulässige Katalysatorkonzentration im Oxidationsprodukt beeinflussen und dass deshalb bei Verwendung solcher Regeneratkatalysatorlösungen Schwierigkeiten und Ausbeuteverluste auftreten, die bei Abwesenheit von TMS und TMME nicht auftreten.

Diese Ergebnisse sind um so überraschender, als in der JA-AS 1239/78 vom 17. Januar 1978 angegeben ist, dass durch Zugabe mehrwertiger Carbonsäuren oder ihrer Anhydride, unter anderem TMS, während der Extraktion von Katalysatorbestandteilen der Oxidation von Alkylaromaten der Übergang von Eisenionen in den Extrakt unterdrückt wird und somit die katalytische Selektivität des Extraktes gesteigert wird; deshalb war mit der vorliegenden Erfindung ein erhebliches technisches Vorurteil zu überwinden.

Weiterhin ist überraschend, dass die Schwierigkeiten und Ausbeuteverluste bei Verwendung TMS-haltiger Katalysatorlösungen bei den technisch bevorzugten Oxidationsbedingungen beobachtet werden, z.B. bei kontinuierlicher Oxidation in Reaktorkaskaden unter 4–8 bar Überdruck, nicht aber bei diskontinuierlicher Oxidation unter Normaldruck, wie sie im Labor zur Katalysatorprüfung üblich ist.

Alle Aufgaben werden erfindungsgemäss dadurch gelöst, dass der Oxidationskatalysator durch Flüssig-Flüssig-Extraktion aus dem oben beschriebenen katalysatorhaltigen Destillationsrückstand gewonnen wird, dass der gegebenenfalls eingeengte Extrakt zur Oxidation von pX und PTE nach dem Witten-Verfahren verwendet wird, dass dabei bestimmte kritische Katalysatorkonzentrationen im Oxidationsprodukt eingestellt werden, die vom Mengenverhältnis zwischen TMS und TMME einerseits und Schwermetalloxidationskatalysator anderseits in der Regeneratkatalysatorlösung abhängen und dass dieses Mengenverhältnis geeignet eingestellt wird.

Die Schwierigkeiten und Ausbeuteverluste können erfindungsgemäss dadurch vermieden werden, dass im Extrakt das Mengenverhältnis $\frac{a}{b}$ von Trimellitsäure plus Trimellitsäuremonomethylester zu Schwermetalloxidationskatalysator auf einen Wert von höchstens 1,8 : 1 eingestellt wird und dass in der Oxidation die in ppm ausgedrückte Katalysatorkonzentration auf $c = 44 \cdot \frac{a}{b} + d$ mit 60 ppm $\leq d \leq$ 300 ppm und dem vorbezeichneten Wert des Verhältnisses $\frac{a}{b}$ eingestellt wird.

Die Katalysatorkonzentration im Oxidationsprodukt wird eingestellt, dass sie nicht niedriger ist als die durch Formel (1) gegebene Mindestkonzentration $c_{min}$:

$$c_{min} = 44 \cdot \frac{a}{b} + 60 \qquad (1),$$

wobei «$c_{min}$» die Mindestkonzentration des Schwermetallkatalysators im Oxidationsprodukt in ppm angibt, während a die Summe der Konzentrationen von TMS und TMME in der Katalysatorlösung in g/l angibt und b die Konzentration des Schwermetallkatalysators in dieser Lösung, ebenfalls in g/l. Der Quotient $\frac{a}{b}$ gibt demnach das Mengenverhältnis von TMS + TMME zu Schwermetalloxidationskatalysator in der Lösung an.

Um die Betriebssicherheit zu erhöhen, sind jedoch Katalysatorkonzentrationen nicht unterhalb der durch Formel (2) gegebenen Mindestkonzentration vorzuziehen, und Katalysatorkonzentrationen nicht unterhalb der durch Formel (3) gegebenen Mindestkonzentration sind besonders vorzuziehen.

$$c_{min} = 44 \cdot \frac{a}{b} + 80 \qquad (2)$$

$$c_{min} = 44 \cdot \frac{a}{b} + 90 \qquad (3)$$

Falls zu hohe Katalysatorkonzentrationen im Oxidationsprodukt eingestellt werden, können die Veresterungskolonnen verstopfen, weiterhin entstehen höhere Rückgewinnungskosten und höhere Katalysatorverluste. Diese Schwierigkeiten können erfindungsgemäss ohne Nachteile vermieden werden, wenn im Oxidationsprodukt Katalysatorkonzentrationen eingestellt werden, die nicht niedriger sind als die durch die Gleichungen (1)–(3) gegebenen Mindestkonzentrationen und nicht höher als die durch Formel (4) gegebene Höchstkonzentration «$c_{max}$», angegeben in ppm. Vorzuziehen sind Katalysatorkonzentrationen nicht oberhalb der durch Formel (5) gegebenen Höchstkonzentration, und besonders vorzuziehen sind Katalysatorkonzentrationen nicht oberhalb der durch Formel (6) gegebenen Höchstkonzentration.

$$c_{max} = 44 \cdot \frac{a}{b} + 300 \qquad (4)$$

$$c_{max} = 44 \cdot \frac{a}{b} + 200 \qquad (5)$$

$$c_{max} = 44 \cdot \frac{a}{b} + 130 \qquad (6)$$

Die erfindungsgemäss im Oxidationsprodukt notwendige Katalysatorkonzentration steigt mit steigendem Gehalt der Regeneratkatalysatorlösung an TMS und TMME, bezogen auf ihren Gehalt an Oxidationskatalysator. Deshalb ist ein hoher Gehalt der Lösung an TMS und TMME, bezogen auf Oxidationskatalysator, unwirtschaftlich. Erfindungsgemäss arbeitet man mit einem Mengenverhältnis von höchstens 1,8 Gramm TMS und TMME pro Gramm Oxidationskatalysator; vorzuziehen sind höchstens 1,0 Gramm TMS und TMME pro Gramm Oxidationskatalysator; noch mehr vorzuziehen sind höchstens 0,8 Gramm TMS und TMME pro Gramm Oxidationskatalysator.

Die Methoden, mit denen diese erfindungsgemässen Mengenverhältnisse von TMS und TMME zu Oxidationskatalysator eingestellt werden, sind nicht kritisch. Diese Mengenverhältnisse können z.B. durch zusätzliche Zugabe löslicher Verbindungen des Schwermetalloxidationskatalysators eingestellt werden. Es kann auch eine Regeneratkatalysatorlösung mit hohem TMS-Gehalt mit einer Katalysatorlösung, die wenig TMS enthält, in geeignetem Verhältnis gemischt werden, so dass eine Lösung mit einer der Erfindung entsprechenden Bemessung resultiert.

Ferner können TMS und TMME aus wässrigen Regeneratkatalysatorlösungen mit schwachbasischen Anionenaustauscherharzen entfernt werden. Besonders gut geeignet sind makroporöse schwachbasische Anionenaustauscherharze in der Acetat- oder Formiatform. Damit werden TMS, TMME und andere in der Katalysatorlösung enthaltene Di- und Tricarbonsäuren gegen Essigsäure oder Ameisensäure ausgetauscht und selektiv aus der Katalysatorlösung entfernt.

Auch können TMS und Trimellitsäureester vor der Extraktion durch Behandlung mit Anionenaustauscherharzen oder durch Destillation aus dem zu extrahierenden Rückstand entfernt werden. Weiterhin ist es möglich, die unerwünschte Umwandlung nicht wasserlöslicher höherer Trimellitsäureester in wasserlösliche TMS und wasserlöslichen TMME durch milde Bedingungen zu verringern, z.B. durch geringe thermische Belastung des Rückstands während der Abtrennung von DMT und anderen Nutzprodukten oder durch niedrige Temperaturen und/oder kurze Verweilzeiten im Extraktor und/oder während einer eventuell auf die Extraktion folgenden Eindampfung der Katalysatorlösung.

Zur Extraktion des Oxidationskatalysators aus den Destillationsrückständen werden zweckmässig verdünnte wässrige Essigsäurelösungen verwendet, insbesondere die essig- und ameisensäurehaltigen Oxidationsabwässer, die bei der Oxidation von PX und PTE nach dem Witten-Verfahren anfallen. Die Extraktion kann nach sämtlichen Extraktionsmethoden durchgeführt werden, z.B. diskontinuierlich oder kontinuierlich, einstufig oder mehrstufig, in Mischer-Absetzern oder in Kolonnen. Die kontinuierliche Extraktion in Mischer-Absetzern wird bevorzugt.

Die Erfindung ist anwendbar auf die für die Oxidation von PX und PTE bekannten Oxidationskatalysatoren, vorzugsweise auf Katalysatoren vom Kobalt-Mangan-Typ, die Kobalt und Mangan in Gewichtsverhältnissen von 40 : 1 bis 2 : 1, besonders vorteilhaft 20 : 1 bis 5 : 1, enthalten.

Die folgenden Beispiele dienen zur weiteren Erläuterung der Erfindung.

Beispiel 1 (Vergleichsbeispiel)

Regeneratkatalysatorlösung wurde aus einem Destillationsrückstand gewonnen, der in einer industriellen Anlage zur DMT-Herstellung nach dem Witten-Verfahren anfiel. In dieser Anlage wurden PX und PTE gemeinsam in flüssiger Phase bei 5–7 bar Überdruck und 150–170 °C Reaktionstemperatur kontinuierlich oxidiert; als Oxidationskatalysator diente eine frisch angesetzte Lösung von Kobaltacetat und Manganacetat in 2%iger wässriger Essigsäure. Diese Lösung wurde kontinuierlich zugegeben, so dass sich im Oxidationsprodukt eine stationäre Konzentration von 90 ppm Kobalt und 9 ppm Mangan einstellte. Das Oxidationsprodukt enthielt neben dem genannten Katalysator und den entstandenen Carbonsäuren nicht umgesetzes Einsatzprodukt und verschiedene Zwischen- und Nebenprodukte. Dieses Oxidationsprodukt wurde bei Temperaturen von etwa 250 °C und Drücken zwischen 20 und 30 bar kontinuierlich mit Methanol verestert. Das Veresterungsprodukt wurde kontinuierlich durch Vakuumdestillation aufgetrennt; diejenigen Bestandteile, die höher als DMT siedeten, wurden unter Vakuum einer thermischen Nachbehandlung unterworfen, wobei weiteres DMT aus den hochsiedenden Bestandteilen abdestillierte.

Gleiche Mengen Destillationsrückstand und Extraktionsmittel wurden kontinuierlich einem mit Rührer versehenen Mischbehälter zugeführt. Die Temperatur des Rückstands betrug 150 °C, die des Extraktionsmittels 50 °C; im Mischbehälter wurde eine Temperatur von 95 °C eingestellt. Als Extraktionsmittel diente Reaktionswasser, das bei der oben beschriebenen industriellen Oxidation von PX und PTE als Brüdenkondensat anfiel und durchschnittlich 2,5 Gewichtsprozent Essigsäure, 1,5 Gewichtsprozent Ameisensäure und 0,8 Gewichtsprozent Formaldehyd enthielt. Durch ein Bodenventil des Mischbehälters wurde kontinuierlich eine Emulsion abgelassen, die in einem Absitzbehälter in eine wässrige und eine organische Phase getrennt wurde. Die wässrige Phase wurde bei Normaldruck eingeengt; etwa ausfallende unlösliche Bestandteile wurden abgetrennt. Die Summe der Konzentrationen von TMS und TMME in der eingeengten Lösung betrug laut polarographischer Analyse 59,3 g/l. Die Kobalt- und die Mangankonzentration wurden titrimetrisch bestimmt. Sie betrugen 35 g/l Kobalt und 3,5 g/l Mangan. Die Lösung enthielt demnach 1,54 g TMS + TMME, bezogen auf ein Gramm Oxidationskatalysator.

Diese Regeneratkatalysatorlösung wurde an Stelle der oben beschriebenen frisch angesetzten Katalysatorlösung kontinuierlich in die industrielle Anlage zur DMT-Herstellung eingesetzt; andere Betriebsgrössen wurden nicht geändert. Nach vier Tagen war die Kohlendioxidkonzentration im Oxidationsabgas auf 3–5 Volumenprozent gestiegen gegenüber 1,8–2,2 vor dem Katalysatorwechsel. Die Kohlenmonoxidkonzentration im Oxidationsabgas stieg gleichzeitig von 0,6–0,8 auf 0,9–1,0 Volumenprozent.

Die xylolbezogene DMT-Ausbeute war bei Verwendung des Regeneratkatalysators 4,5 Molprozent niedriger als bei Verwendung des frisch angesetzten Katalysators.

Beispiel 2 (Vergleichsbeispiel)

Die im Beispiel 1 beschriebene Regeneratkatalysatorlösung wurde in einem kontinuierlich betriebenen Versuchsreaktor aus nichtrostendem Stahl ausgeprüft. Als Indikator für Selektivitätsänderungen dienten die Konzentrationen an Kohlenmonoxid und Kohlendioxid im Oxidationsabgas. Falls die Oxidationsgeschwindigkeit konstant ist, sind diese Konzentrationen direkt als Selektivitätsmass geeignet, weil die ausbeutevermindernden Nebenreaktionen überwiegend zu Kohlenmonoxid und Kohlendioxid führen und weil die Rückstandsbildung in einem positiven Zusammenhang steht mit der Bildung von Kohlenmonoxid und Kohlendioxid. Konstante Oxidationsgeschwindigkeit wurde dadurch erreicht, dass dem Reaktor mit konstanter Geschwindigkeit Luft zugeführt wurde und dass die Oxidationsbedingungen so gewählt wurden, dass der Sauerstoff stets vollständig absorbiert wurde.

Der Versuchsreaktor hatte einen inneren Durchmesser von 40 cm und ein nutzbares Volumen von 1,8 m³; er war ausgerüstet mit einem Lufteinleitungsrohr, einem Doppelmantel zur Heizung oder Kühlung, einem Wasserabscheider, Mess- und Regelvorrichtungen zur Einspeisung von PX und von PTE, Ablassvorrichtungen sowie Dosierpumpen zur Einspeisung von Katalysatorlösung. In den Kopf dieses Reaktors wurde kontinuierlich die in Beispiel 1 beschriebene frisch aus Kobalt- und Manganacetat angesetzte Katalysatorlösung gepumpt sowie 80 kg PX und 93 kg PTE pro Stunde. PX und PTE stammten aus den Fahrtanks der in Beispiel 1 beschriebenen technischen DMT-Anlage. Es wurde so viel Katalysatorlösung eingesetzt, dass sich im Oxidationsprodukt eine stationäre Konzentration von 90 ppm Kobalt und 9 ppm Mangan einstellte. Diese Konzentration wurde durch regelmässige Entnahme und Analyse von Proben überprüft. Mit einer Standregelung wurde durch ein Bodenventil kontinuierlich Flüssigkeit aus dem Versuchsreaktor abgelassen, so dass der Flüssigkeitsinhalt des Reaktors auf 1,7 m³ gehalten wurde. Die Temperatur im Reaktorsumpf wurde durch eine Temperaturregelung auf 159 °C gehalten. Am Boden des Reaktors wurden kontinuierlich 60 Nm³ Luft pro Stunde eingeleitet. Durch eine Druckregelung wurde über ein Entspannungsventil aus dem Reaktorkopf kontinuierlich Gas abgelassen, so dass sich im Gasraum des Reaktors ein Überdruck von 6 bar einstellte. Diese Reaktionsbedingungen entsprechen denen im ersten Reaktor einer dreistufigen technischen Reaktorkaskase.

Die mit dem Oxidationsabgas austretenden kondensierbaren Anteile wurden in einem Kühler kondensiert; danach wurden die Konzentrationen an Sauerstoff, Kohlenmonoxid und Kohlendioxid im Oxidationsabgas kontinuierlich gemessen. Nach eintägigem Betrieb enthielt das Oxidationsabgas 0,6 Volumenprozent Kohlenmonoxid, 1,8 Volumenprozent Kohlendioxid und weniger als 0,1 Volumenprozent Sauerstoff. Diese Abgaszusammensetzung änderte sich nicht innerhalb von fünf Tagen kontinuierlichen Betriebs. Nach diesen fünf Tagen wurde statt der Frischkatalysatorlösung die in Beispiel 1 beschriebene Regeneratkatalysatorlösung kontinuierlich in den Reaktorkopf gepumpt. Die Kobalt- und Mangankonzentrationen im Oxidationsprodukt wurden weiterhin auf 90 ppm Kobalt und 9 ppm Mangan gehalten. Nach der Umstellung auf die Regeneratkatalysatorlösung nahmen die Konzentrationen an Kohlenmonoxid und Kohlendioxid im Abgas langsam zu. Sie betrugen vier Tage nach der Umstellung 0,8 Volumenprozent Kohlenmonoxid und 3,5 Volumenprozent Kohlendioxid.

Beispiel 3

Aus der in Beispiel 1 beschriebenen Regeneratkatalysatorlösung wurden TMS und TMME durch Adsorption an schwach basischem Anionenaustauscherharz entfernt. Dazu wurde Harz mit dem Handelsnamen «Lewatit MP 62» der Firma Bayer AG in Leverkusen in einer Säule mit vierprozentiger Natriumhydroxidlösung aktiviert; danach wurde die Säule mit dem in Beispiel 1 beschriebenen essig- und ameisensäurehaltigen Reaktionswasser aus der industriellen Oxidation von PX und PTE gespült und dadurch das Harz mit Acetat- und Formiationen beladen. Anschliessend wurde die Regeneratkatalysatorlösung bei 60 °C oben auf die Säule gegeben und unten abgezogen. Das Eluat enthielt die Katalysatorlösung und Reste des Spülwassers. Es wurde eingeengt, bis die Kobaltkonzentration wieder 35,0 g/l betrug; daneben enthielt die eingeengte Lösung 3,5 g/l Mangan und weniger als 0,1 g/l TMS und TMME.

Diese Lösung wurde wie in Beispiel 2 beschrieben ausgeprüft. Dabei ergab sich nach Umstellung von Frischkatalysatorlösung auf diese von TMS und TMME befreite Regeneratkatalysatorlösung keine Veränderung der Abgaszusammensetzung. Nach fünf Tagen kontinuierlichen Betriebs mit dem von TMS und TMME befreiten Regeneratkatalysator enthielt das Abgas unverändert 0,6 Volumenprozent Kohlenmonoxid, 1,8 Volumenprozent Kohlendioxid und weniger als 0,1 Volumenprozent Sauerstoff.

Beispiel 4

Die in Beispiel 1 beschriebene TMS-haltige Regeneratkatalysatorlösung wurde in verschiedenen Verhältnissen mit der in Beispiel 3 beschriebenen Regeneratkatalysatorlösung gemischt, aus der TMS und TMME entfernt worden waren. Es resultierten verschiedene Regeneratkatalysatorlösungen, die alle 35 g/l Kobalt und 3,5 g/l Mangan

enthielten, jedoch verschiedene Konzentrationen an TMS und TMME. Diese Lösungen wurden analog Beispiel 2 im kontinuierlich betriebenen Versuchsreaktor ausgeprüft, jedoch wurden zunächst mit Frischkatalysator Konzentrationen von 300 ppm Kobalt und 30 ppm Mangan im Reaktionsprodukt eingestellt. Dann wurde auf Regeneratkatalysator umgestellt und weiterhin bei einer Katalysatorkonzentration von 300 ppm Kobalt und 30 ppm Mangan oxidiert. Unter diesen Bedingungen änderte sich die Zusammensetzung des Oxidationsabgases nicht; das Abgas enthielt unverändert 0,6 Volumenprozent Kohlenmonoxid, 1,8 Volumenprozent Kohlendioxid und weniger als 0,1 Volumenprozent Sauerstoff. Jetzt wurde die Katalysatordosierung langsam in kleinen Schritten verringert; die Katalysatorkonzentration im Oxidationsprodukt wurde regelmässig bestimmt, und die Abgaszusammensetzung wurde kontinuierlich gemessen. Die Sauerstoffkonzentration im Oxidationsabgas lag stets unterhalb von 0,1 Volumenprozent, die Kohlendioxidkonzentration begann bei Unterschreitung bestimmter kritischer Katalysatorkonzentrationen anzusteigen. Als geringste zulässige Katalysatorkonzentrationen wurden diejenigen festgelegt, bei denen die Kohlendioxidkonzentration im Abgas 2,1 Volumenprozent betrug. Diese geringsten zulässigen Katalysatorkonzentrationen waren abhängig vom Mengenverhältnis zwischen TMS + TMME einerseits und Schwermetalloxidationskatalysator anderseits in der Regeneratkatalysatorlösung. Tabelle 1 zeigt diese Abhängigkeit.

Tabelle 1

| Beispiel Nr. | Mengenverhältnis $\frac{a}{b}$ in der Katalysatorlösung $\left[\frac{g\ (TMS\ +\ TMME)}{g\ Katalysator}\right]$ | Katalysatorkonzentration im Oxidationsprodukt bei 2,1 Vol.-% $CO_2$ im Abgas $\left[ppm\ (Co\ +\ Mn)\right]$ |
|---|---|---|
| 4.1 | 0 | 73 |
| 4.2 | 0,40 | 82 |
| 4.3 | 0,88 | 100 |
| 4.4 | 1,02 | 110 |
| 4.5 | 1,20 | 116 |
| 4.6 | 1,54 | 132 |

**Patentansprüche**

1. Verfahren zur Gewinnung und Wiederverwendung von Schwermetalloxidationskatalysator durch Extraktion von hochsiedenden Destillationsrückständen, die bei der Oxidation von p-Xylol und/oder p-Toluylsäuremethylester enthaltenden Gemischen in flüssiger Phase mit Sauerstoff oder einem Sauerstoff enthaltenden Gas bei erhöhtem Druck und erhöhter Temperatur in Gegenwart von gelöstem Schwermetalloxidationskatalysator, anschliessenden Veresterung des Oxidationsprodukts mit Methanol bei erhöhtem Druck und erhöhter Temperatur und destillativen Auftrennung des Veresterungsprodukts in eine Roh-DMT-Fraktion, eine p-toluylsäuremethylesterreiche Fraktion und einen hochsiedenden Destillationsrückstand ohne Kontaktbehandlung dieses Rückstandes mit Methanol anfallen, mit Wasser oder verdünnten wässrigen Lösungen niedermolekularer aliphatischer Monocarbonsäuren oder Alkohole und Rückführung – ohne Methanolbehandlung – des Extraktes, ggf. nach Einengen, in die Oxidation, dadurch gekennzeichnet, dass im Extrakt das Mengenverhältnis $\frac{a}{b}$ von Trimellitsäure plus Trimellitsäuremonomethylester zu Schwermetalloxidationskatalysator auf einen Wert von höchstens 1,8 : 1 eingestellt wird und dass in der Oxidation die in ppm ausgedrückte Katalysatorkonzentration auf $c = 44 \cdot \frac{a}{b} + d$ mit 60 ppm $\leq d \leq$ 300 ppm und dem vorbezeichneten Wert des Verhältnisses $\frac{a}{b}$ eingestellt wird.

2. Verfahren nach Anspruch 1, dadurch gekennzeichnet, dass die Konzentration an Trimellitsäure plus Trimellitsäuremonomethylester im Extrakt durch Ionenaustausch mit der Acetat- und/oder Formiatform schwachbasischer Ionenaustauscherharze verringert wird.

### Claims

1. Process for the recovery and reuse of heavy metal oxidation catalyst by extraction from high boiling distillation residues which accumulate in the oxidation of p-xylene and/or p-toluic acid methyl ester-containing mixtures in the liquid phase with oxygen or an oxygen-containing gas at elevated pressure and elevated temperature in the presence of dissolved heavy metal oxidation catalyst, subsequent esterification of the oxidation product with methanol at elevated pressure and elevated temperature and distillative separation of the esterification product into a crude DMT-fraction, a p-toluic acid methyl ester-rich fraction and a high boiling distillation residue, without contacting of this residue with methanol, with water or dilute aqueous solutions of low molecular aliphatic monocarboxylic acids or alcohols, and feedback – without methanol treatment – of the extract, optionally after concentration, into the oxidation, characterised in that, in the extract, the mass ratio $\frac{a}{b}$ of trimellitic acid plus trimellitic acid monomethyl ester to heavy metal oxidation catalyst is employed at a value of, at the most, 1.8 : 1 and that in the oxidation the catalyst concentration, expressed in ppm, is established at $c = 44 \cdot \frac{a}{b} + d$, with 60 ppm $\leq d \leq$ 300 ppm and the above designated value of the ratio $\frac{a}{b}$.

2. Process according to claim 1, characterised in that the concentration of trimellitic acid plus trimellitic acid monomethyl ester in the extract is reduced by ion exchange with the acetate and/or formate form of weakly basic ion exchange resins.

### Revendications

1. Procédé de récupération et de réutilisation d'un catalyseur d'oxydation à base de métal lourd, par extraction de résidus de distillation à point élevé d'ébullition que l'on obtient lors de l'oxydation en phase liquide de mélanges contenant du p-xylène et/ou de l'ester méthylique de l'acide toluylique par de l'oxygène ou par un gaz contenant de l'oxygène sous pression élevée et à température élevée en présence d'un catalyseur d'oxydation à base d'un métal lourd dissous, puis estérification du produit d'oxydation par du méthanol sous pression élevée et à température élevée et séparation par distillation du produit d'estérification en une fraction de téréphtalate de diméthyle brut, une fraction riche en ester méthylique de l'acide p-toluylique et un résidu de distillation à point élevé d'ébullition sans soumettre ce résidu en contact avec du méthanol, avec de l'eau ou avec des solutions aqueuses diluées d'acides monocarboxyliques ou d'alcools aliphatiques à bas poids moléculaire et recyclage, sans traitement par du méthanol, de l'extrait, éventuellement après concentration, vers l'oxydation, caractérisé en ce qu'on ajuste dans l'extrait le rapport a/b des quantités de l'acide trimellitique plus l'ester monométhylique de l'acide trimellitique au catalyseur d'oxydation à base de métal lourd à une valeur d'au maximum 1,8 : 1 et en ce qu'on ajuste dans l'oxydation la concentration du catalyseur, exprimée en ppm, à $c = 44 \cdot \frac{a}{b} + d$, avec 60 ppm $\leq d \leq$ 300 ppm et avec la valeur indiquée ci-dessus du rapport a/b.

2. Procédé selon la revendication 1, caractérisé en ce qu'on diminue dans l'extrait la concentration de l'acide trimellitique plus l'ester monométhylique de l'acide trimellitique par échange d'ions avec des résines d'échange d'ions faiblement basiques sous forme acétate et/ou formiate.